(19) Europäisches Patentamt
European Patent Office
Office européen des brevets

(11) Veröffentlichungsnummer: **0 227 983**
**A1**

(12) # EUROPÄISCHE PATENTANMELDUNG

(21) Anmeldenummer: **86117014.0**

(22) Anmeldetag: **08.12.86**

(51) Int. Cl.[4]: **C 07 D 251/50**

(30) Priorität: **20.12.85 DE 3545460**

(43) Veröffentlichungstag der Anmeldung: **08.07.87 Patentblatt 87/28**

(84) Benannte Vertragsstaaten: **CH DE FR GB LI**

(71) Anmelder: **BAYER AG, Konzernverwaltung RP Patentabteilung, D-5090 Leverkusen 1 Bayerwerk (DE)**

(72) Erfinder: **Jäger, Horst, Dr., Carl-Rumpff-Strasse 37, D-5090 Leverkusen (DE)**

(54) **Verfahren zur Herstellung von Fluortriazinylverbindungen.**

(57) Ein verbessertes Verfahren zur Herstellung von Verbindungen der Formel

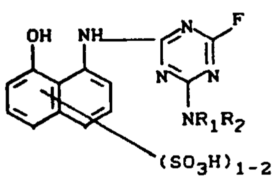

(1)

worin
$R_1$ und $R_2$ = Wasserstoff, gegebenenfalls substituiertes Alkyl, Cycloalkyl oder Aryl, wobei $R_1$ und $R_2$ gegebenenfalls unter Einschluß eines Heteroatoms einen Ring bilden können, besteht darin, daß man die Kondensation des Aminoaphthols mit dem Trifluortriazin und/oder die Kondensation des dabei erhältlichen Reaktionsproduktes mit den entsprechenden Aminen in Gegenwart von Borsäure oder deren Salzen durchführt.

EP 0 227 983 A1

0227983

BAYER AKTIENGESELLSCHAFT 5090 Leverkusen, Bayerwerk
Konzernverwaltung RP
Patentabteilung My/li-c

## Verfahren zur Herstellung von Fluortriazinylverbindungen

Gegenstand der vorliegenden Erfindung ist ein verbessertes Verfahren zur Herstellung von Verbindungen der Formel

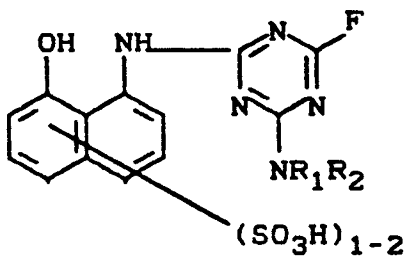

(1)

worin

$R_1$ und $R_2$ = Wasserstoff, gegebenenfalls substituiertes Alkyl, Cycloalkyl oder Aryl, wobei $R_1$ und $R_2$ gegebenenfalls unter Einschluß eines Heteroatoms einen Ring bilden können,

durch Umsetzung von Verbindungen der Formel

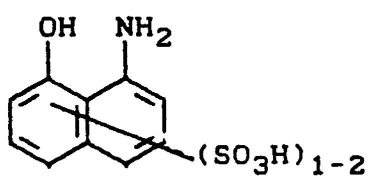

(2)

mit Trifluortriazin (3) zu Verbindungen der Formel

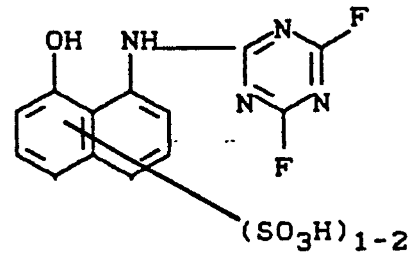

(4)

und anschließende Umsetzung mit Aminen der Formel

$$HNR_1R_2 \quad (5)$$

in wäßrigem Medium, dadurch gekennzeichnet, daß man die Umsetzung von (2) mit (3) und/oder (4) mit (5) in Gegenwart von Borsäure oder deren Salzen durchführt.

Pro Mol der Verbindung (2) bzw. (4) verwendet man 0,2 bis 2,0 Mol Borsäure oder deren Salze, vorzugsweise 0,8 bis 1,2 Mol.

Geeignete Salze der Borsäure sind insbesondere die Alkalisalze (Li, Na, K).

Geeignete Alkylreste $R_1$ und $R_2$ sind beispielsweise $C_1$-$C_4$-Alkyl, gegebenenfalls substituiert durch OH, $C_1$-$C_4$-Alkoxy oder Phenyl, gegebenenfalls weiter substituiert, beispielsweise durch Sulfo.

Geeignete Arylreste $R_1$ und $R_2$ sind beispielsweise Phenyl, gegebenenfalls substituiert durch Sulfo, $C_1$-$C_4$-Alkyl, $C_1$-$C_4$-Alkoxy, Halogen, Carboxy.

Ein geeigneter Cycloalkylrest ist beispielsweise Cyclohexyl. Bevorzugt ist die Herstellung von Verbindungen der Formel

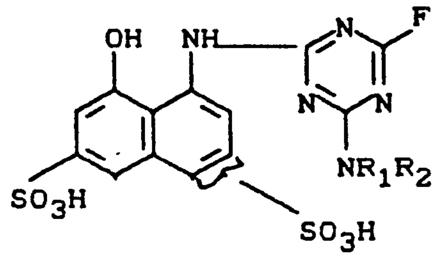

(6)

insbesondere von solchen der Formel (6), in denen

$R_1$ = H, $C_1$-$C_4$-Alkyl
$R_2$ = gegebenenfalls durch $C_1$-$C_4$-Alkyl, bspw. $CH_3$, $C_2H_5$, durch $C_1$-$C_4$-Alkoxy, bspw. $CH_3O$, $C_2H_5O$, $HOC_2H_4O$, Halogen, wie Cl, Br, F, COOH, und/oder Sulfo substituiertes Phenyl.

Verbindungen der Formel (4) erhält man beispielsweise nach den Angaben der Deutschen Offenlegungsschrift 2 747 011 aus Aminonaphtholsulfosäuren (2) durch Kondensation mit 2,4,6-Trifluortriazin.

Beispiele für Verbindungen der Formel (2) sind folgende:
1-Amino-8-hydroxynaphthalin-3,6-disulfonsäure
1-Amino-8-hydroxynaphthalin-4,6-disulfonsäure
1-Amino-8-hydroxynaphthalin-3,5-disulfonsäure
1-Amino-8-hydroxynaphthalin-4-sulfonsäure.

Beispiele für Amine der Formel (5) sind insbesondere die auf Seiten 26 und 27 der EP-A 131545 genannten.

Die Umsetzung von (2) mit (3) bzw. (4) mit (5) erfolgt unter den in der DE-A 27 47 011 angegebenen Temperatur- und pH-Bedingungen, wobei als alkalisch wirkende Mittel insbesondere LiOH, $Li_2CO_3$, $NaHCO_3$, $Na_2CO_3$, NaOH, $KHCO_3$

$K_2CO_3$, KOH, $MgCO_3$, $Mg(OH)_2$, $CaCO_3$, $Ca(OH)_2$ verwendet werden.

Beispiel 1

0,5 mol 1-Amino-8-hydroxynaphthalin-3,6-disulfonsäure (H-Säure) werden in 850 ml Wasser mit 50 ml 40 volumen%iger Natronlauge gelöst; pH 6,0. Man setzt 500 g Eis zu und läßt dann in 20 Minuten 45 ml 2,4,6-Trifluortriazin einlaufen, wobei man zunächst den pH auf 3,8 bis 4 abfallen läßt und ihn dann durch Eintropfen von 15%iger Sodalösung in diesem Bereich hält. Nach beendeter Zugabe rührt man noch 5 Minuten nach und überzeugt sich durch eine Titration von der Vollständigkeit der Acylierung. Volumen 1700 ml. Temp. $0^0$ C. In die klare Lösung gibt man 34,5 g Borsäure (0,5 Mol) und anschließend 46,5 g Anilin, wobei die bei der Kondensation freiwerdende Flußsäure mit 15%iger Sodalösung so neutralisiert wird, daß der pH der Lösung bei 5,0 bis 5,5 gehalten wird. Man rührt noch 30 Minuten nach, wobei die Temperatur von $0^0$ auf 10 - $15^0$C ansteigt. Im Dünnschichtchromatogramm erkennt man, daß sich das Difluortriazin vollständig umgesetzt hat.

Die Umsetzung verläuft in der gleichen Weise, wenn man anstelle von H-Säure 1-Amino-8-hydroxynaphthalin-4,6-disulfonsäure (K-Säure) und anstelle Anilin eines der nachfolgend genannten Amine verwendet:
o-Toulidin, p-Toluidin, o-Anisidin, p-Anisidin, o-Chloranilin, m-Chloranilin, p-Chloranilin, o-Phenetidin, p-Phenetidin, p-ß-Hydroxyethoxyanilin, o-ß-Hydroxyethoxyanilin, N-Methylanilin, N-Ethylanilin, m-Sulfanilsäure, o-, p-Sulfanilsäure, 4-Methoxyanilin-2-sulfonsäure, 2-Aminonaphthalin-6-sulfonsäure.

0227983

Beispiel 2

0,5 mol 1-Amino-8-hydroxynaphthalin-3,6-disulfonsäure (H-Säure) werden in 850 ml Wasser mit 100 ml 11 volumen%iger Lithiumhydroxidlösung gelöst, pH 6,0. Man setzt 500 g Eis und 34,5 g Borsäure (0,5 mol) zu und läßt dann in 20 Minuten 45 ml 2,4,6-Trifluor-triazin einlaufen, wobei man zunächst den pH auf 3,8 bis 4 abfallen läßt und ihn dann durch Eintropfen von 11 %iger Lithiumhydroxidlösung in diesem Bereich hält. Nach beendeter Zugabe rührt man noch 5 Minuten nach und überzeugt sich durch eine Titration von der Vollständigkeit der Acylierung. Volumen 1700 ml. Temp. $0^{0}$ C. In die klare Lösung gibt man 46,5 g Anilin, wobei die bei der Kondensation freiwerdende Flußsäure mit 11 %iger Lithiumhydroxidlösung so neutralisiert wird, daß der pH der Lösung bei 5-5,5 gehalten wird. Man rührt noch 30 Minuten nach, wobei die Temperatur von $0^{0}$C auf 10-$15^{0}$ C ansteigt. Im Dünnschichtchromatogramm erkennt man, daß sich das Trifluortriazin vollständig umgesetzt hat.

Die Umsetzung verläuft in der gleichen Weise, wenn man anstelle von H-Säure 1-Amino-8-hydroxynaphthalin-4,6-disulfonsäure (K-Säure) und anstelle Anilin eines der nachfolgend genannten Amine verwendet:
o-Toluidin, p-Toluidin, o-Anisidin, p-Anisidin, o-Chloranilin, m-Chloranilin, p-Chloranilin, o-Phenetidin, p-Phenetidin, p-ß-Hydroxyethoxyanilin, o-ß-Hydroxyethoxyanilin, N-Methylanilin, N-Ethylanilin, m-Sulfanilsäure, o-, p-Sulfanilsäure, 4-Methoxyanilin-2-sulfonsäure, 2-Aminonaphthalin-6-sulfonsäure.

Patentansprüche

1. Verfahren zur Herstellung von Verbindungen der Formel

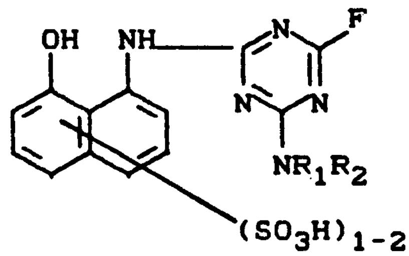

(1)

worin

$R_1$ und $R_2$ = Wasserstoff, gegebenenfalls substituiertes Alkyl, Cycloalkyl oder Aryl, wobei $R_1$ und $R_2$ gegebenenfalls unter Einschluß eines Heteroatoms einen Ring bilden können,

durch Umsetzung von Verbindungen der Formel

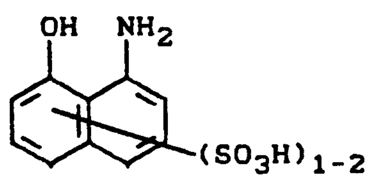

(2)

mit Trifluortriazin (3) zu Verbindungen der Formel

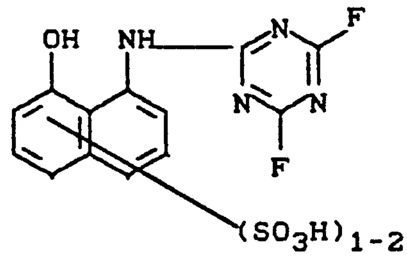

(4)

und anschließende Umsetzung mit Aminen der Formel

$HNR_1R_2$ (5)

in wäßrigem Medium, dadurch gekennzeichnet, daß man die Umsetzung von (2) mit (3) und/oder (4) mit (5) in Gegenwart von Borsäure oder deren Salzen durchführt.

2. Verfahren nach Anspruch 1, dadurch gekennzeichnet, daß man pro Mol (2) bzw. (4) 0,2-2 Mol Borsäure oder deren Salze verwendet.

3. Verfahren nach Anspruch 2, dadurch gekennzeichnet, daß man 0,8-1,2 Mol Borsäure oder deren Salze pro Mol (2) bzw. (4) verwendet.

Europäisches Patentamt

# EUROPÄISCHER RECHERCHENBERICHT

0227983
Nummer der Anmeldung

EP 86 11 7014

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 014 844 (BAYER AG)<br>* Anspruch 3 *<br>--- | 1 | C 07 D 251/50 |
| A | DE-A-2 729 240 (BAYER AG)<br>* Anspruch 10 *<br>--- | 1 | |
| D,A | DE-A-2 747 011 (CIBA-GEIGY AG)<br>* Ansprüche 1-3 *<br>--- | 1 | |
| A | EP-A-0 036 133 (HOECHST AG)<br>* Ansprüche 1, 4 *<br>--- | 1 | |
| D,A | EP-A-0 131 545 (CIBA-GEIGY AG)<br>----- | | |

| RECHERCHIERTE SACHGEBIETE (Int. Cl.4) |
|---|
| C 07 D 251/00 |

Der vorliegende Recherchenbericht wurde für alle Patentansprüche erstellt.

| Recherchenort | Abschlußdatum der Recherche | Prüfer |
|---|---|---|
| BERLIN | 05-03-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument